# EUROPÄISCHE PATENTANMELDUNG

(11) **EP 0 768 066 A2**
(43) Veröffentlichungstag der Anmeldung: **16.04.1997**
(21) Anmeldenummer: 96115614.8
(22) Anmeldetag: 28.09.1996
(51) Int. Cl.: A61F 2/36, A61L 27/00

(54) **Hüftkopfkappe für eine Hüftgelenkprothese**

(30) Priorität: 10.10.1995 DE 19537676
(71) Anmelder: CERASIV GmbH INNOVATIVES KERAMIK-ENGINEERING, D-73207 Plochingen (DE)
(72) Erfinder: Hoch, Ernst, 73274 Notzingen (DE); Pfaff, Hans-Georg, 73760 Ostfildern (DE)
(74) Vertreter: Scherzberg, Andreas, Dr.

(57) **Zusammenfassung**

Zur besseren und zementfreien Verankerung einer Hüftkopfkappe für eine Hüftgelenkprothese wird vorgeschlagen,daß der Kugelkopf (1) aus Keramik gefertigt ist und daß zwischen dem Kugelkopf (1) und dem Femurkopf ein Metallteil (2,8,9,10) angeordnet ist.

## Beschreibung

Die Erfindung betrifft eine Hüftkopfkappe für eine Hüftgelenkprothese nach dem Oberbegriff des Anspruchs 1.

Aus der DE-OS 25 12 407 ist eine Hüftkopfkappe für eine Hüftgelenkprothese bekannt, die im wesentlichen die Form einer Hohlkugelfläche hat. Diese Hüftkopfkappe ist mit Knochenzement auf dem Gelenkkopf des Oberschenkelknochens befestigt. Damit überflüssiger Knochenzement besser abfließen kann, ist im Scheitelpunkt der Hüftkopfkappe eine radiale Bohrung angeordnet.

Nachteilig an der Fixation mit Knochenzement ist, daß es leicht zu Lockerungen der Hüftkopfkappe auf dem Femurkopf bzw. dem Gelenkkopf des Oberschenkelknochens kommt. Dies hat dann eine erneute Operation und eine Schaftimplantation zur Folge.

Der Erfindung liegt die Aufgabe zugrunde, eine Hüftkopfkappe für eine Hüftgelenkprothese nach dem Oberbegriff des Anspruchs 1 derart zu verbessern, daß ohne Verwendung von Knochenzement eine bessere und dauerhaftere Fixation der Hüftkopfkappe auf dem Femurkopf erreicht ist.

Erfindungsgemäß wird diese Aufgabe dadurch gelöst, daß der Kugelkopf aus Keramik ist und daß zwischen dem Kugelkopf und dem Femurteil ein Metallteil angeordnet ist.

Durch dieses Metallteil zwischen dem Femurkopf und dem Kugelkopf ist eine dauerhafte und stabile Befestigung ohne Knochenzement zu erreichen. Der Kugelkopf ist aus Keramik gefertigt. Keramik zeichnet sich durch besonders gute Gleiteigenschaften gegenüber natürlichen Gelenkflächen und Polyethylenen aus.

In einer bevorzugten Ausführungsform ist die Öffnung im Kugelkopf als konische Bohrung ausgeführt und das Metallteil hat eine an diese konische Bohrung angepaßte Außenfläche. Die Befestigung kann dann vorteilhafterweise z.B. mittels konischer Klemmung erfolgen. Der Winkel der konischen Klemmung liegt vorteilhafterweise zwischen 1° und 18°.

In einer besonders bevorzugten Ausführungsform ist die Öffnung im Kugelkopf als konische Bohrung und das Metallteil als konisch geformte Hülse ausgebildet, wobei die Hülse einen sich über die gesamte Länge erstreckenden Spalt aufweist, so daß sich beim Einsetzen des Metallteils in den Kugelkopf eine Spreizwirkung ergibt, die das Metallteil im Kugelkopf hält.

Zur verbesserten Verankerung weist die Öffnung im Kugelkopf vorteilhafterweise eine zum Femurkopf hin gerichtete Einschnürung auf. Diese Einschnürung verhindert ein Herausgleiten des Metallteils aus der Öffnung im Kugelkopf.

In besonders vorteilhafter Ausgestaltung weist das Metallteil an seiner zum Femurkopf gerichteten Innenseite ein Gewinde auf, welches in den Femurkopf eingeschnitten wird. Hierzu ist das Gewinde zweckmäßigerweise ein Schneidgewinde. Bevorzugt erstreckt sich das Gewinde über die gesamte Länge des Metallteils.

Erfindungsgemäß besteht das Metallteil bevorzugt aus Titan oder einer Titanlegierung.

In einer alternativen Ausführungsform weist das Metallteil eine zylindrische Kontur auf und ist durch Presspassung, durch Schrumpfpassung oder durch eine Spreizkonstruktion in den Kugelkopf eingefügt.

Die Innenoberfläche des Metallteils weist vorteilhafterweise eine Struktur auf. Diese Struktur kann z.B. durch Sandstrahlen oder durch Eindrehen von Rillen geschaffen werden. Vorteilhafterweise besteht die Struktur auch aus durch elektrische Erosion eingebrachten Makroporen. Die Innenoberfläche kann natürlich auch glatt sein und keine Struktur aufweisen.

Zweckmäßigerweise ist die Innenoberfläche des Metallteils mit einer bioaktiven Keramik, vorzugsweise Hydroxylapatit oder einer porösen Metallbeschichtung versehen. Dies kann mit der beschriebenen Struktur kombiniert werden.

Alternativ ist die Öffnung des Kugelkopfes mit einer metallischen Beschichtung, vorzugsweise einer porösen Metallbeschichtung versehen. Diese Metallbeschichtung bildet dann das Metallteil zwischen Femurkopf und Kugelkopf.

Weitere Merkmale ergeben sich aus den Figuren, die nachfolgend eingehend beschrieben werden. Es zeigt:
- Fig. 1: einen Kugelkopf einer erfindungsgemäßen Hüftkopfkappe,
- Fig. 2: denselben Kugelkopf wie in Fig. 1, jedoch mit eingesetztem Metallteil mit Innengewinde,
- Fig. 3: einen ähnlichen Kugelkopf wie Fig. 2, jedoch mit einem Metallteil ohne Gewinde,
- Fig. 4: einen Kugelkopf mit einem zylindrischen Metallteil und
- Fig. 5: einen Kugelkopf mit metallischer Beschichtung der Öffnung.

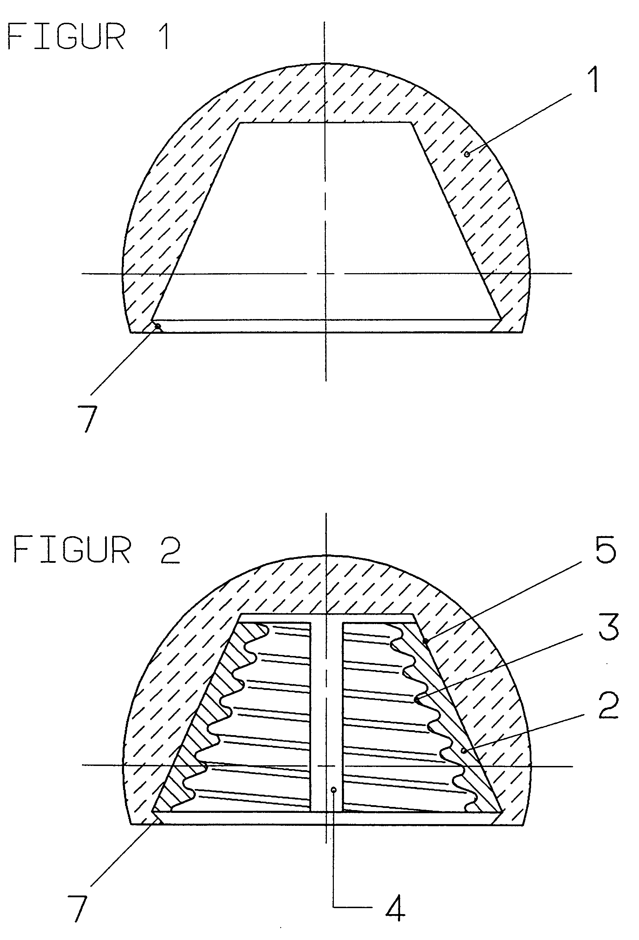

Fig. 1 zeigt einen Kugelkopf 1 einer erfindungsgemäßen Hüftkopfkappe für eine Hüftgelenkprothese. Der Kugelkopf 1 weist eine Öffnung 6 auf, die als konische Bohrung ausgeführt ist. An der zum nicht gezeigten Femurkopf hin gerichteten Seite der Öffnung ist eine umlaufende Einschnürung 7 angeordnet. Nicht gezeigt ist, daß der Kugelkopf 1 in eine Hüftgelenkpfanne eingebettet ist. Der Kugelkopf 1 besteht aus einer Keramik.

Fig. 2 zeigt denselben Kugelkopf 1 wie in Fig. 1, nur ist hier ein Metallteil 2 in den Kugelkopf 1 eingesetzt. Das Metallteil 2 ist eine konisch geformte Hülse mit einem auf seiner Innenseite angeordneten Schneidgewinde 3, welches sich über die gesamte Länge des Metallteils 2 erstreckt. Das Metallteil 2 weist einen sich über die gesamte Länge des Metallteils 2 erstreckenden Spalt 4 auf, so daß das Metallteil 2 über eine Klemmwirkung im Kugelkopf 1 gehalten ist. Die Einschnürung 7 bewirkt eine axiale Fixierung. Das Metallteil 2 besteht aus einer Titanlegierung und ist auf seiner Innenseite mit Hydroxylapatit oder einem anderen bioaktiven Material beschichtet.

Nicht gezeigt ist, daß das Metallteil 2 über sein Gewinde 3 in den Femurkopf eingeschnitten ist.

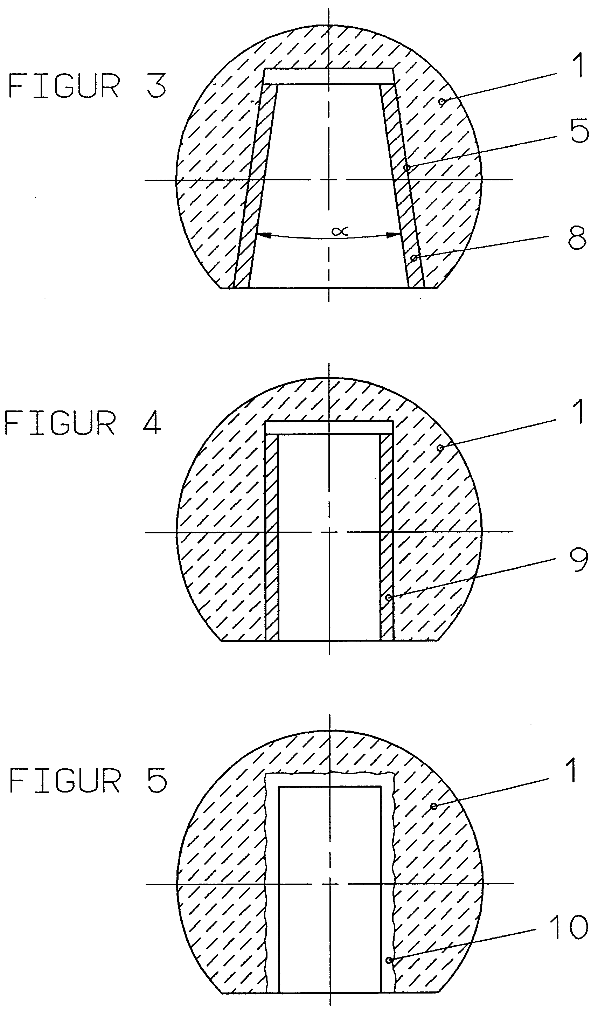

Fig. 3 zeigt einen Kugelkopf 1 mit einem Metallteil 8, welches durch eine konische Klemmung 5 im Kugelkopf 1 fixiert ist. Der Winkel α der konischen Klemmung 5 liegt dabei zwischen 1° und 18°.

Fig. 4 zeigt eine Ausführungsform mit einer zylindrischen Öffnung im Kugelkopf 1 und einem Metallteil 9 mit einer zylindrischen Kontur. Das Metallteil 9 ist durch Presspassung oder durch Schrumpfpassung in den Kugelkopf 1 eingefügt worden.

In Fig. 5 weist der Kugelkopf 1 ebenfalls eine zylindrische Öffnung auf. Die Oberfläche der Öffnung ist hier jedoch mit einer metallischen Beschichtung 10 versehen. Diese Beschichtung ersetzt die Funktion des Metallteils und ist bervorzugt eine poröse Titanbeschichtung.

## Patentansprüche

1. Hüftkopfkappe für eine Hüftgelenkprothese mit einem Kugelkopf (1), der auf einen präparierten Femurkopf aufgesetzt ist, wobei der Kugelkopf (1) eine Öffnung (6) aufweist, **dadurch gekennzeichnet**,
- daß der Kugelkopf (1) aus Keramik gefertigt ist und
- daß zwischen dem Kugelkopf (1) und dem Femurkopf ein Metallteil (2,8,9,10) angeordnet ist.

2. Hüftkopfkappe nach Anspruch 1, **dadurch gekennzeichnet**, daß der Kugelkopf (1) mittels konischer Klemmung (5) auf dem Metallteil (2,8) befestigt ist.

3. Hüftkopfkappe nach Anspruch 2, **dadurch gekennzeichnet**, daß der Winkel der konischen Klemmung (5) zwischen 1° und 18° liegt.

4. Hüftkopfkappe nach einem der Ansprüche 1 bis 3, **dadurch gekennzeichnet**, daß das Metallteil (2,8) als konisch geformte Hülse ausgebildet ist.

5. Hüftkopfkappe nach Anspruch 4, **dadurch gekennzeichnet**, daß die Hülse einen sich über die gesamte Länge erstreckenden Spalt (4) aufweist, so daß sich beim Einsetzen in den Kugelkopf (1) eine Spreizwirkung der Hülse ergibt.

6. Hüftkopfkappe nach Anspruch 5, **dadurch gekennzeichnet**, daß die Öffnung (6) im Kugelkopf (1) eine zum Femurkopf hin gerichtete Einschnürung (7) aufweist.

7. Hüftkopfkappe nach einem der Ansprüche 1 bis 6, **dadurch gekennzeichnet**, daß das Metallteil (2) an seiner zum Femurkopf gerichteten Innenseite ein Gewinde (3) aufweist, welches in den Femurkopf eingeschnitten wird.

8. Hüftkopfkappe nach Anspruch 7, **dadurch gekennzeichnet**, daß das Gewinde (3) des Metallteils (2) ein Schneidgewinde ist.

9. Hüftkopfkappe nach Anspruch 7 oder 8, **dadurch gekennzeichnet**, daß sich das Gewinde (3) über die gesamte Länge des Metallteils (2) erstreckt.

10. Hüftkopfkappe nach einem der Ansprüche 1 bis 9, **dadurch gekennzeichnet**, daß das Metallteil (2,8,9,10) aus Titan oder einer Titanlegierung besteht.

11. Hüftkopfkappe nach einem der Ansprüche 1 bis 10, **dadurch gekennzeichnet**, daß das Metallteil (9) eine zylindrische Kontur aufweist und durch Presspassung, durch Schrumpfpassung oder durch eine Spreizkonstruktion in den Kugelkopf (1) eingefügt ist.

12. Hüftkopfkappe nach einem der Ansprüche 1 bis 11, **dadurch gekennzeichnet**, daß die Innenoberfläche des Metallteils (2,8,9,10) eine Struktur aufweist.

13. Hüftkopfkappe nach einem der Ansprüche 1 bis 12, **dadurch gekennzeichnet**, daß die Innenoberfläche des Metallteils (2,8,9,10) mit einer bioaktiven Keramik, vorzugsweise Hydroxylapatit, oder einer porösen Metallbeschichtung versehen ist.

14. Hüftkopfkappe nach einem der Ansprüche 1 bis 13, **dadurch gekennzeichnet**, daß die Öffnung (6) des Kugelkopfes (1) mit einer metallischen Beschichtung (10), vorzugsweise einer porösen Titanbeschichtung, versehen ist.
